Europäisches Patentamt

European Patent Office

Office européen des brevets

(11) Veröffentlichungsnummer : **0 086 879**
**B1**

(12) **EUROPÄISCHE PATENTSCHRIFT**

(45) Veröffentlichungstag der Patentschrift :
18.09.85

(51) Int. Cl.⁴ : **A 61 F   2/30**

(21) Anmeldenummer : 82110497.3

(22) Anmeldetag : 13.11.82

(54) Zentrierende Halterung für einen in seiner Längsachse verschiebbaren Schaft einer in einen Röhrenknochen einsetzbaren Endoprothese.

(30) Priorität : 23.02.82 CH 1094/82

(43) Veröffentlichungstag der Anmeldung :
31.08.83 Patentblatt 83/35

(45) Bekanntmachung des Hinweises auf die Patenterteilung : 18.09.85 Patentblatt 85/38

(84) Benannte Vertragsstaaten :
AT BE DE FR GB IT NL

(56) Entgegenhaltungen :
DE-A- 2 103 640
DE-B- 1 625 394
DE-B- 2 247 560
DE-B- 2 338 136
FR-A-   897 568
FR-A- 2 502 939
GB-A- 2 065 819
US-A- 4 011 602

(73) Patentinhaber : GEBRÜDER SULZER AKTIENGE-
SELLSCHAFT
Zürcherstrasse 9
CH-8401 Winterthur (CH)

(72) Erfinder : Frey, Otto
Wallrütistrasse 56
CH-8404 Winterthur (CH)

(74) Vertreter : Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)

**Beschreibung**

Die Erfindung betrifft eine zentrierende Halterung für einen in seiner Längsachse verschiebbaren Schaft einer in einen Röhrenknochen einsetzbaren Endoprothese, bestehend aus einer aufspreizbaren Verankerungshülse und mindestens einem in der Verankerungshülse in Achsrichtung verstellbaren, aussen konischen Spreizkörper, sowie aus einer Führung für den mindestens im Bereich der Führung im Querschnitt invarianten Schaft, die als an den Schaft angepasster Gleit-Pass-Sitz ausgebildet ist.

Verankerungsschäfte von Endoprothesen, beispielsweise von Kniegelenkprothesen, sollten in vielen Fällen so im Knochen gelagert werden, dass sie in axialer Richtung verschiebbar, in radialer Richtung jedoch zentriert sind. Zusätzlich tritt dabei häufig die Komplikation auf, dass die Abstützung des Schaftes, die im allgemeinen auf dem kortikalen Gewebe des Knochens erfolgt, in einem sich in axialer Richtung relativ stark verengenden oder in einem sich vom äusseren Ende her erweiternden Markhohlraum erfolgen muss. Dadurch wird die Fixierung einer zentrierenden Halterung im Knochen erschwert.

Aus der DE-A-23 38 136 ist eine Halterung der genannten Art bekannt ; diese ist für Kleingelenke, z. B. Fingergelenke, bestimmt. Sie besteht aus einem, an den Schaft im Gleit-Pass-Sitz angepassten Köcher, in den das Schaftende eingeschoben ist. In der axialen Verlängerung des Schaftendes ist das Verankerungselement mit Hilfe eines Spreizkörpers zur Fixierung im Knochen aufspreizbar. Diese Halterung hat den Nachteil, dass sie nicht an einer beliebigen Stelle der Schaftlänge im Knochen fixiert werden kann ; darüberhinaus sind bei ihr Führungsweg und-/oder Verankerungslänge sehr oft begrenzt und daher ungenügend.

Aufgabe der Erfindung ist es, eine Halterung für einen, axial beweglichen, jedoch radial zentrierten Schaft zu schaffen, die selbst in jedem beliebigen Bereich von im Querschnitt etwa konstanten oder sich axial erweiternden bzw. verengenden Markhohlräumen sicher fixierbar ist und darüberhinaus ausreichend lange Führungswege und Verankerungslängen aufweist.

Gemäss der vorliegenden Erfindung wird diese Aufgabe dadurch gelöst, dass für eine Verankerungshülse zwei in axialem Abstand zueinander angeordnete Spreizkörper vorgesehen sind, die beide je mit einem Teilhohlraum der Führung versehen sind.

Der Schaftquerschnitt kann beliebig — d. h. beispielsweise kreisförmig, elliptisch oder oval — ausgebildet sein. Die Verlegung der Führung in die Spreizkörper und die Aufteilung der Halterung in zwei Spreizkörper und eine diese aufnehmende Verankerungshülse ermöglicht eine Fixierung der Halterung in jedem beliebigen axialen Bereich des Röhrenknochens ; dazu wird die Verankerungshülse in dessen Markhohlraum eingeführt, an einer gewünschten Stelle festgehalten und mit Hilfe der in Achsrichtung verschiebbaren Spreizkörper · längs mindestens zweier, im axialen Abstand voneinander liegender Bereiche ihres Umfangs aufgespreizt und fixiert. Die Fixierung der Verankerungshülse in zwei Bereichen der axialen Länge bietet eine hohe Gewähr gegen unbeabsichtigte Verschiebungen der Halterung — besonders auch bei im wesentlichen zylindrischen, d. h. sich nicht stark verengenden oder erweiternden Markhohlräumen — während der « Lebensdauer » der Prothese.

Die Fixierung der Halterung und/oder die Verankerung des Schaftes können sowohl völlig knochenzementfrei als auch in einem zusätzlichen, als Füllung des Markhohlraumes dienenden Zementbett erfolgen. Weiterhin können die eine Aufspreizung bewirkenden Konen an den Spreizkörpern unter sich und gegenüber denjenigen in der Verankerungshülse gleiche oder verschiedene Steigungen haben ; ebenso können die Durchmesser der zur gleichen Verankerungshülse gehörenden Spreizkörper verschieden gross sein. Durch unterschiedliche Grössen der Spreizkörper und/oder unterschiedliche Konussteigungen der Spreizkörper und der « zugehörigen » Teilabschnitte des Innenhohlraumes der Verankerungshülse wird — insbesondere bei stark « konischen » Markhohlräumen — eine verschieden starke Aufspreizung der beiden Enden der Hülse erleichtert.

Im einfachsten Fall werden die Spreizkörper in den Innenhohlraum der Verankerungshülse hineingezogen oder hineingeschlagen, wobei widerhakenartige Verzahnungen oder andere geeignete Oberflächenstrukturen am Aussenmantel der Spreizkörper und/oder im Innenhohlraum der Verankerungshülse das gegenseitige Ineinandergreifen verbessern und die Verbindung beider Teile gegen ein unbeabsichtigtes Lösen sichern können ; der Innenhohlraum der Verankerungshülse kann dabei einen zylindrischen und/oder einen mit zwei axial zueinander versetzten konischen Teilabschnitten versehenen Querschnitt haben.

Um ein Aufspreizen der Verankerungshülse zu erleichtern, ist es vorteilhaft, diese mit im wesentlichen in Achsrichtung verlaufenden Längsschlitzen zu versehen und/oder als einen verformbaren Hohlzylinder zu gestalten, der längs seines Umfangs wellenförmig ausgebildet ist.

Werden an die Genauigkeit, mit der die Halterung in einem bestimmten axialen Bereich des Knochens fixiert sein soll, hohe Anforderungen gestellt, so ist es zweckmässig, wenn die Spreizkörper in die Verankerungshülse einschraubbar sind, da dann weniger die Gefahr besteht, dass die Verankerungshülse infolge eines axialen Zuges oder Druckes verschoben wird.

Unterschiedlich starke Aufspreizungen an beiden Enden der Verankerungshülse lassen sich auch erreichen, wenn die Konen der Spreizkörper gleich gerichtet in die Verankerungshülse eingezogen werden und ihre Durchmesser verschieden sind.

Sind starke Aufspreizungen der Verankerungshülse erforderlich, so ist es zweckmässig, wenn die Spreizkörper in mindestens zwei konzentrisch ineinander gesteckte Konusteile unterteilt sind, von denen der äussere aufspreizbar ist ; dazu ist es vorteilhaft, den äusseren Konusteil mit mindestens im wesentlichen in Achsrichtung verlaufenden Längsschlitzen zu versehen. Weiterhin können die beiden Konusteile eines Spreizkörpers durch geeignete Oberflächenstrukturen gegenseitig gegen ein ungewolltes Lösen gesichert werden. Ebenso lässt sich die Haftung der Verankerungshülse im Knochen verbessern, wenn ihre äussere Mantelfläche mit einer Oberflächenstruktur, beispielsweise mit noppenartigen Vorsprüngen oder Vertiefungen bzw. Löchern, versehen wird.

Als Materialien für die neue Halterung sind alle in der Implantat-Technik üblichen Werkstoffe geeignet ; vorzugsweise wird für beide Einzelteile der Halterung Kunststoff, insbesondere Polyäthylen der Klassifikationen HDPE und UHMW verwendet. Weiterhin ist es möglich, die Oberflächen mindestens teilweise mit Beschichtungen zu versehen, die beispielsweise die Reibung des Schaftes in dem Gleit-Pass-Sitz erniedrigen — zwischen der Fixierung dienenden Oberflächen jedoch erhöhen — oder das Anwachsen von Gewebe fördern.

Im folgenden wird die Erfindung anhand von Ausführungsbeispielen im Zusammenhang mit der Zeichnung näher erläutert.

Figur 1 zeigt schematisch ein erstes Ausführungsbeispiel der neuen Halterung, bei dem die im Schnitt dargestellten Spreizkörper noch nicht in die Verankerungshülse eingezogen ist ;

Figur 2 ist eine Ansicht in Richtung A von Figur 1 ;

Figur 3 ist ein Schnitt III-III von Figur 1 ;

Figur 4 gibt eine in einen Röhrenknochen fixierte Halterung wieder, in der ein Schaft gelagert ist ;

Figur 5 zeigt im Längsschnitt eine zweite Ausführungsform ;

Figur 6 gibt in gleicher Darstellung ein drittes Ausführungsbeispiel wieder ;

Figur 7 stellt — teilweise in Ansicht und teilweise im Längsschnitt — eine vierte Ausführungsform der Halterung dar, wobei

Figur 8 eine Aufsicht auf Figur 7 in Richtung des Pfeiles B ist.

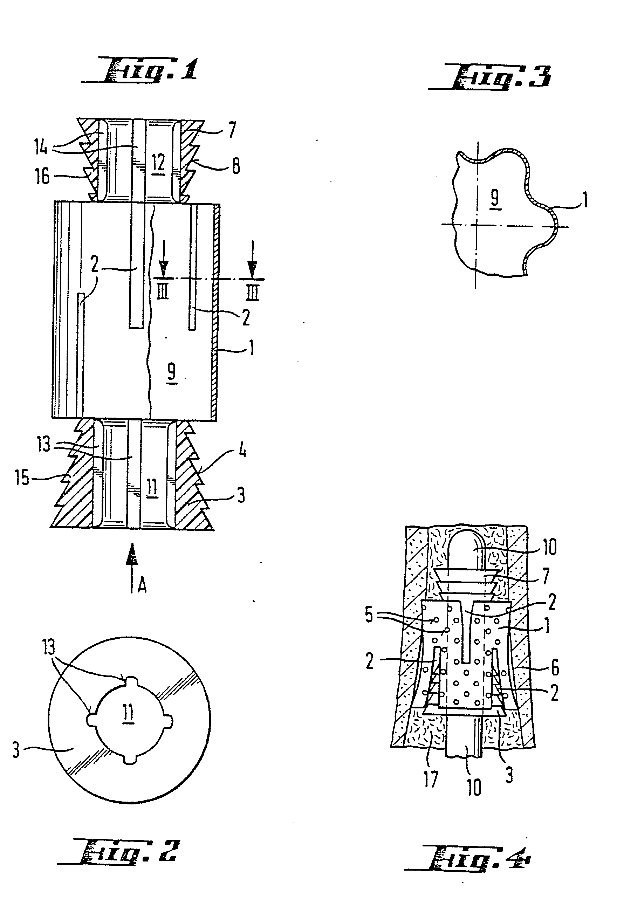

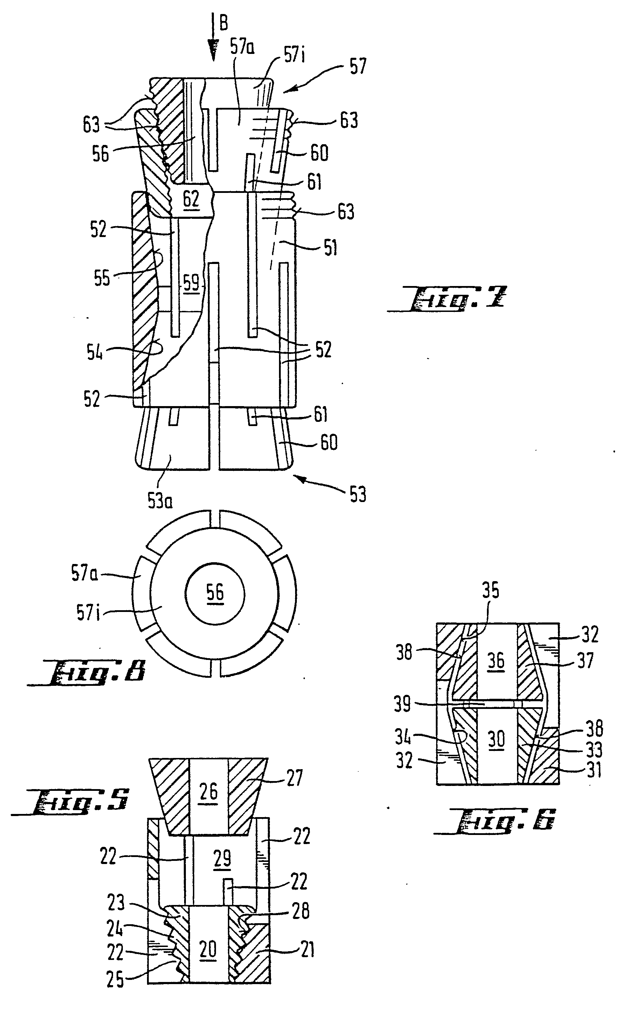

Die Halterung nach Figur 1 umfasst eine Verankerungshülse 1, die einen wellenförmig gefalteten Mantel (Figur 3) aufweist und aus dünnem elastisch und plastisch verformbaren Material, beispielsweise aus Blech, besteht. Von beiden Enden her sind über den Umfang verteilt Längsschlitze 2 in der Verankerungshülse 1 vorhanden, die das Aufspreizen der Hülse 1 durch von beiden Seiten einziehbare Spreizkörper 3 und 7 mit konischen Aussenmänteln 4 und 8 erleichtern. Die äussere Oberfläche der Verankerungshülse 1 kann mit einer Oberflächenstruktur, z. B. mit Noppen oder Löchern 5 (Figur 4), versehen sein, durch die ihre Haftung im kortikalen Knochengewebe 6 (Figur 4) verbessert wird. Wie Figur 1 und 3 zeigen, hat bei diesem Ausführungsbeispiel der Innenhohlraum 9 der Verankerungshülse 1 eine kreiszylindrische Grundform.

Für die Aufnahme eines Prothesenschaftes 10 (Figur 4) im Gleit-Pass-Sitz sind die Hohlräume 11 bzw. 12 der Spreizkörper 3 und 7 an den im gezeigten Beispiel kreiszylindrischen Schaftquerschnitt in Form und Abmessungen angepasst ; Längsnuten 13 und 14 in den Wänden der Hohlräume 11 und 12 dienen für den Eingriff von Werkzeugen, mit denen die Spreizkörper 3 und 7 in die Verankerungshülse 1 hineingezogen, -gedrückt oder -geschraubt werden können.

Auf ihren konischen Aussenmänteln 4 und 8 sind die im Querschnitt kreisförmigen (Figur 2) Spreizkörper 3 und 7 mit einer Verzahnung 15 und 16 versehen, die ihren Sitz in der Verankerungshülse 1 verbessern. Wie ein Vergleich der in Figur 1 gezeigten Spreizkörper 3 und 7 deutlich macht, sind beide unterschiedlich gross. Dadurch wird ein verschieden starkes Aufspreizen der Verankerungshülse 1 erreicht, wenn diese, wie in Figur 4 gezeigt, in einem sich konisch verengenden Markhohlraum 17 fixiert werden muss.

Zur Einführung in den Markhohlraum 17 (Figur 4) eines Knochens 6, der sich nach oben verengt, werden die Spreizkörper 3 und 7 und die Verankerungshülse 1 an nicht dargestellten Werkzeugen befestigt und gemeinsam oder einzeln in den markhohlraum 17 eingeführt. Im vorgesehenen axialen Abstand — der beispielsweise durch eine Markierung am Haltewerkzeug für die Verankerungshülse 1 festgelegt sein kann — vom Rand des geöffneten Knochens 6 hält man nun die Verankerungshülse 1 fest und zieht mit Hilfe weiterer Werkzeuge die Spreizkörper 3 und 7 so weit in die Verankerungshülse 1 hinein, dass der mit Schlitzen 2 versehene Mantel mit einer ausreichenden Kraft gegen das Kortikale Knochengewebe 6 gepresst wird, um eine sichere Fixierung der Halterung zu gewährleisten ; wie Figur. 4 zeigt, können dabei die Eindringtiefen der beiden Spreizkörper 3 und 7 in die Hülse 1 verschieden sein. Nach Lösen der Werkzeuge von der Halterung kann der Schaft 10 in die Hohlräume 11 und 12 der Spreizkörper 3 und 7 eingeschoben werden (Figur 4).

Bei dem Ausführungsbeispiel nach Figur 5 ist die Verankerungshülse 21 in ihrer Grundform wiederum ein Hohlzylinder, der über einen gewissen Bereich seiner axialen Höhe von beiden Enden her mit über den Umfang verteilten Längsschlitzen 22 versehen ist, die sein Aufspreizen erleichtern bzw. ermöglichen. Ihr Innenhohlraum 29 ist im oberen Bereich zylindrisch und verengt sich im unteren Drittel seiner axialen Länge konisch, wobei im konischen Bereich ein Gewinde 25 vorgesehen ist, in das ein entsprechendes Gegengewinde 24 auf dem konischen Aussenmantel eines im Innern der Verankerungshülse 21 angeordneten ersten Spreizkörper 23 eingeschraubt ist.

Dieser Spreizkörper 23 dient zur Spreizung des unteren Endes der Verankerungshülse 21, indem er mit seinem konischen Aussenmantel tiefer in den konischen Teil des Innenhohlraumes 29 hineingeschraubt wird. Ein zweiter Spreizkörper 27, dessen konischer Aussenmantel in der gleichen Richtung wie derjenige des Spreizkörpers 23 verläuft, jedoch eine andere Steigung besitzt, ist von oben in den zylindrischen Teil des Innenhohlraumes 29 einschiebbar, um das obere Ende der Verankerungshülse 21 aufzuspreizen. Neben unterschiedlichen Steigungen der Aussenmäntel sind auch die Durchmesser der Spreizkörper 23 und 27 verschieden, so dass unterschiedlich starke Spreizungen erreicht werden können. Zur Aufnahme eines Prothesenschaftes sind in den Spreizkörper 23 und 27 Hohlräume 20 und 26 vorgesehen, die wiederum an den aufzunehmenden Schaft angepasst sind.

Bei dem Ausführungsbeispiel nach Fig. 6 enthält der Innenhohlraum 39 der mit Längsschlitzen 32 versehenen Verankerungshülse 31 zwei gegenläufig konische Teilabschnitte 34 und 35 ; die beiden zugehörigen Spreizkörper 33 und 37, die in diesem Beispiel gleiche Form und Abmessungen haben, sind im Innern des Innenhohlraumes 39 gelagert und bewirken ein Aufspreizen der Verankerungshülse 31 dadurch, dass ihre konischen Aussenmäntel 38 in die sich nach aussen verjüngenden konischen Teilabschnitte 34 und 35 der Verankerungshülse 31 hineingepresst werden. Die Hohlräume für die Aufnahme des Schaftes sind in diesem Beispiel mit 30 und 36 bezeichnet. Die Ausführungsform der Erfindung nach Fig. 6 hat den Vorteil, dass die Halterung als Ganzes, also als ein Teil, in den Markhohlraum des Knochens eingeführt werden kann.

Selbstverständlich sind auch bei den Ausführungsbeispielen der Halterung nach Fig. 5 und 6 geeignete, nicht ausdrücklich dargestellte Massnahmen für den Eingriff von Werkzeugen oder Instrumenten getroffen, mit deren Hilfe die Halterungen in den Knochen eingeführt und in diesem fixiert werden.

Bei der Verankerungshülse 51 des letzten Ausführungsbeispiels nach Fig. 7 und 8, deren Längsschlitze mit 52 bezeichnet sind, besitzt der Innenhohlraum 59 wiederum zwei konische Teilabschnitte 54 und 55, deren Konensteigungen unterschiedlich sind und die sich von den beiden Enden her gegen die Mitte verjüngen. In diese Teilabschnitte 54 und 55 sind die Spreizkörper 53 und 57 einziehbar, wodurch die Enden der Verankerungshülse 51 aufgespreizt werden.

Die Spreizkörper 53 und 57 sind selbst jedoch in einen äusseren und einen inneren Konusteil 57a und 57i unterteilt, was nur an dem oberen Spreizkörper 57 dargestellt ist. Der innere Konusteil 57i, der den Führungshohlraum 56 für den nicht dargestellten Schaft enthält, ist seinerseits in den äusseren Konusteil 57a hineinziehbar. Der äussere Konusteil 57a — und entsprechend der äussere Konusteil 53a — sind dabei von beiden Enden her mit Längsschlitzen 0 und 61 versehen, durch die ihre Aufweitung erleichtert bzw. ermöglicht wird.

Mit dieser Konstruktion, die vornehmlich verwendet wird, wenn starke Aufspreizungen der Verankerungshülse 51 erforderlich sind, wird nicht nur ein stufenweises Aufspreizen erreicht, sondern zusätzlich auch ermöglicht, dass die Verankerungshülse 51 nicht nur an ihren Enden, sondern auch in mittleren Bereichen aufgeweitet werden kann. Diesem Zweck dienen die inneren Längsschlitze 61 der äusseren Konusteile 53a und 57a ; die inneren Enden dieser Konusteile 53a und 57a werden dabei aufgespreizt, indem der innere Konusteil 57i mindestens so tief in den Innenholraum 62 des äusseren Konusteils 57a hineingezogen wird, dass der Durchmesser seines auf der Höhe des inneren Endes des äusseren Konusteils 57a befindlichen Aussenmantelquerschnitts grösser ist als die unbelastete lichte Weite des inneren Endes des Hohlraumes 62.

Die Oberflächenstrukturen 63 auf den Aussenmänteln der verschiedenen Konusteile bzw. der Verankerungshülse 51 und/oder in den Begrenzungen der verschiedenen Innenhohlräume 59 und 62 dienen wiederum als Sicherungen gegen ungewolltes Lösen der konischen Verbindungen oder ungewolltes Verschieben der Verankerungshülse 51, nachdem diese im Knochen fixiert worden sind.

**Patentansprüche**

1. Zentrierende Halterung für einen in seiner Längsachse verschiebbaren Schaft (10) einer in einen Röhrenknochen (6) einsetzbare Endoprothese, bestehend aus einer aufspreizbaren Verankerungshülse (1, 21, 31, 51) und mindestens einem in der Verankerungshülse in Achsrichtung verstellbaren, aussen konischen Spreizkörper (3, 7 ; 23, 27 ; 33, 37 ; 53, 57), sowie aus einer Führung (11, 12 ; 20, 26 ; 30, 36 ; 56) für den mindestens im Bereich der Führung im Querschnitt invarianten Schaft (10), die als an den Schaft angepasster Gleit-Pass-Sitz ausgebildet ist, dadurch gekennzeichnet, dass für eine Verankerungshülse (1, 21, 31, 51) zwei in axialem Abstand zueinander angeordnete Spreizkörper (3, 7 ; 23, 27 ; 33, 37 ; 53, 57) vorgesehen sind, die beide je mit einem Teilhohlraum (11, 12 ; 20, 26 ; 30 ; 56) der Führung versehen sind.

2. Halterung nach Anspruch 1, dadurch gekennzeichnet, dass der Innenhohlraum (29 ; 39 ; 59) der Verankerungshülse (21 ; 31 ; 51) mindestens einen konischen Teilabschnitt (28 ; 34, 35 ; 54, 55) hat.

3. Halterung nach Anspruch 1, dadurch gekennzeichnet, dass die Verankerungshülse (1 ; 21 ; 31 ; 51) mit über den Umfang verteilten Längsschlitzen (2 ; 22 ; 32 ; 52) versehen ist.

4. Halterung nach Anspruch 1, dadurch gekennzeichnet, dass die Verankerungshülse (1) aus einem verformbaren Hohlzylinder besteht, der längs seines Umfangs wellenförmig ausgebildet ist (Fig. 3).

5. Halterung nach Anspruch 1, dadurch gekennzeichnet, dass ein Spreizkörper (53, 57) in mindestens zwei konzentrisch ineinander gesteckte Konusteile (53a, 57a, 57i) unterteilt ist, von denen der äussere (53a, 57a) aufspreizbar ist.

6. Halterung nach Anspruch 5, dadurch gekennzeichnet, dass der äussere Konusteil (53a, 57a) mit mindestens im wesentlichen in Achsrichtung verlaufenden Längsschlitzen (60, 61) versehen ist.

7. Halterung nach Anspruch 2, dadurch gekennzeichnet, dass die Konen der Innenhohlraum-Teilabschnitte (34, 35 ; 54, 55) der Verankerungshülse (21, 31, 51) gegenläufig zueinander angeordnet sind.

8. Halterung nach Anspruch 7, dadurch gekennzeichnet, dass die gegenläufigen Konen der Teilabschnitte (54, 55) der Verankerungshülse (51) unterschiedliche Steigung haben.

9. Halterung nach Anspruch 1, dadurch gekennzeichnet, dass die Spreizkörper (3, 7 ; 23, 27), die einer Verankerungshülse (1 ; 21) zugeordnet sind, unterschiedliche Durchmesser und/oder unterschiedliche Konussteigungen haben.

10. Halterung nach Anspruch 2, dadurch gekennzeichnet, dass ein konischer Teilabschnitt (55) des Innenhohlraums (59) einer Verankerungshülse (51) und der zugehörige Spreizkörper (57) unterschiedliche Konussteigungen haben.

11. Halterung nach Anspruch 1, dadurch gekennzeichnet, dass mindestens einer (23) der Spreizkörper (23, 27) in die Verankerungshülse (21) einschraubbar ist.

## Claims

1. Centring retention for a longitudinally displaceable stem (10) of an endoprosthesis introducible into a medullated bone (6), the retention comprising an expanding anchorage sleeve (1, 21, 31, 51) and at least one externally conical expanding member axially adjustable therein, and a guide for the stem which is of invariable cross-section at least near the guide, the same taking the form of a sliding-to-snug-fit seat adapted to the stem, characterised in that two axially spaced-apart expanding members (3, 7 ; 23, 27 ; 33, 37 ; 53, 57) are provided for one anchorage sleeve (1, 21, 31, 51) and are each formed with a partial cavity (11, 12 ; 20, 26 ; 30 ; 56) of the guide.

2. Retention according to claim 1, characterised in that the inner cavity (29 ; 39 ; 59) of the anchorage sleeve (21 ; 31 ; 51) has at least one conical portion (28 ; 34, 35 ; 54, 55).

3. Retention according to claim 1, characterised in that the anchorage sleeve (1 ; 21 ; 31 ; 51) is formed over its periphery with longitudinal slots (2 ; 22 ; 32 ; 52).

4. Retention according to claim 1, characterised in that the anchorage sleeve (1) is in the form of a deformable hollow cylinder of wavy shape along its periphery (Fig. 3).

5. Retention according to claim 1, characterised in that an expanding member (53, 57) is subdivided into at least two cone parts (53a, 57a, 57i) engaged concentrically one in another, the outer such part (53a, 57a) being expandable.

6. Retention according to claim 5, characterised in that the outer cone part (53a, 57a) is formed with at least substantially axial longitudinal slots (60, 61).

7. Retention according to claim 2, characterised in that the cones of the internal cavity parts (34, 35 ; 54, 55) of the anchorage sleeve (21, 31, 51) are disposed in opposite directions to one another.

8. Retention according to claim 7, characterised in that the oppositely directed cones of the internal cavity parts (54, 55) of the anchorage sleeve (51) are at different inclinations from one another.

9. Retention according to claim 1, characterised in that the expanding members (3, 7 ; 23, 27) associated with an anchorage sleeve (1, 21) are of different diameters and/or different cone inclinations from one another.

10. Retention according to claim 2, characterised in that a conical part (55) of the internal cavity (59) of a anchorage sleeve (51) has a different cone inclination from the associated expanding member (57).

11. Retention according to claim 1, characterised in that at least one (23) of the expanding members (23, 27) can be screwed into the anchorage sleeve (21).

## Revendications

1. Support à effet de centrage pour la tige (10), mobile longitudinalement, d'une endoprothèse pouvant être implantée dans un os long (6), comprenant une douille d'ancrage (1, 21, 31, 51) pouvant être écartée et au moins un organe d'écartement (3, 7 ; 23, 27 ; 33, 37 ; 53, 57) extérieurement tronconique et pouvant être ajusté dans le sens axial de la douille d'ancrage, ainsi qu'un système de guidage (11, 12 ; 20, 26 ; 30, 36 ; 56) de la tige (10) présentant une section constante au moins au voisinage de ce système de guidage réalisé sous la forme d'une assise coulissante adaptée à ladite tige, caractérisé par le fait qu'on a prévu, pour une douille d'ancrage (1, 21, 31, 51), deux organes d'écartement (3, 7 ; 23, 27 ; 33, 37 ; 53, 57) disposés à distance axiale l'un de l'autre et munis l'un et l'autre d'un logement partiel respectif (11, 12 ; 20, 26 ; 30 ; 56) du système de guidage.

2. Support selon la revendication 1, caractérisé par le fait que l'espace interne creux (29 ; 39 ; 59) de la douille d'ancrage (21 ; 31 ; 51) présente au moins une zone partielle tronconique (28 ; 34, 35 ; 54, 55).

3. Support selon la revendication 1, caractérisé par le fait que la douille d'ancrage (1 ; 21 ;

31 ; 51) est dotée de fentes longitudinales (2 ; 22 ; 32 ; 52) réparties sur le pourtour.

4. Support selon la revendication 1, caractérisé par le fait que la douille d'ancrage (1) consiste en un cylindre creux déformable, de réalisation ondulée le long de son pourtour (figure 3).

5. Support selon la revendication 1, caractérisé par le fait qu'un organe d'écartement (53, 57) est scindé en au moins deux parties tronconiques (53a, 57a, 57i) emboîtées concentriquement l'une dans l'autre, parmi lesquelles la partie externe (53a, 57a) peut être écartée.

6. Support selon la revendication 5, caractérisé par le fait que la partie tronconique externe (53a, 57a) est dotée de fentes longitudinales (60, 61) s'étendant au moins sensiblement dans le sens axial.

7. Support selon la revendication 2, caractérisé par le fait que les cônes des zones partielles (34, 35 ; 54, 55) de l'espace interne creux de la douille d'ancrage (21, 31, 51) sont disposés dans des directions mutuellement opposées.

8. Support selon la revendication 7, caractérisé par le fait que les cônes opposés des zones partielles (54, 55) de la douille d'ancrage (51) présentent une inclinaison différente.

9. Support selon la revendication 1, caractérisé par le fait que les organes d'écartement (3, 7 ; 23, 27), associés à une douille d'ancrage (1 ; 21), présentent des diamètres différents et/ou des inclinaisons de conicité différentes.

10. Support selon la revendication 2, caractérisé par le fait qu'une zone partielle tronconique (55) de l'espace interne creux (59) d'une douille d'ancrage (51) et l'organe d'écartement associé (57) présentent des inclinaisons de conicité différentes.

11. Support selon la revendication 1, caractérisé par le fait qu'au moins l'un (23) des organes d'écartement (23, 27) peut être vissé dans la douille d'ancrage (21).

**Fig. 1**

**Fig. 3**

**Fig. 2**

**Fig. 4**

*Fig. 7*

*Fig. 8*

*Fig. 6*

*Fig. 5*